# EUROPEAN PATENT APPLICATION

(11) **EP 3 048 104 A1**
(43) Date of publication of application: **27.07.2016**
(21) Application number: 15151854.5
(22) Date of filing: 20.01.2015
(51) Int. Cl.: C07D 473/34, A61K 31/52

(54) **AMORPHOUS AND CRYSTALLINE FORMS OF IDELALISIB AND PROCESS FOR FORMING THE SAME**

(71) Applicant: Sandoz AG, 4056 Basel (CH)
(72) Inventor: The designation of the inventor has not yet been filed
(74) Representative: Englmeier, Ludwig

(57) **Abstract**

The present invention concerns pure and stable amorphous idelalisib, crystalline solvates of idelalisib and a process for the formation of pure and stable amorphous idelalisib using the crystalline solvates of idelalisib.

## Description

The present invention concerns amorphous idelalisib, crystalline solvates of idelalisib and a process for the preparation of amorphous idelalisib using the crystalline solvates of idelalisib.

### Background of the Invention

An important property of a medicament for oral use is the bioavailability of the active pharmaceutical ingredient (API). Bioavailability refers to the extent and rate at which the active moiety (drug or metabolite) enters systemic circulation, thereby accessing the site of action. One important factor influencing the bioavailability of a medicament is the solubility of an API, in particular in those cases where the API has a low solubility and a high permeability.

It is known that different solid forms, e.g. different polymorphs, of an active pharmaceutical ingredient can have a different solubility. When such different polymorphic forms of an API are administered to the patient they might result in different in-vivo profiles. Further, different polymorphic forms might convert into each other during production of the dosage form or during storage. As a result, dosage forms containing more than one polymorphic forms of the API might have the drawback of showing unpredictable in-vivo profiles.

Furthermore, an at least partial change of the degree of structural order of a pressure-sensitive solid form of the active pharmaceutical ingredient during the production of a compressed solid dosage form can also have negative implications for the relevant properties of the active pharmaceutical ingredient, such as compactability or compatibility with other excipients, thus impeding the process for the preparation of the compressed solid dosage form. In summary, there are various reasons why the use of pure solid forms of an API in pharmaceutical compositions is generally preferred over the use of solid form mixtures.

Idelalisib is a compound of the general formula (1) 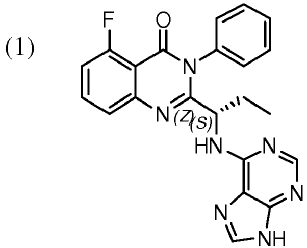 and has the IUPAC name (S)-2-(1-(9H-purin-6-ylamino) propyl)-5-fluoro-3-phenylquinazolin-4(3H)-one and is alternatively referred to as 5-fluoro-3-phenyl-2-[(1S)-1-(9H-purin-6-ylamino)propyl]quinazolin-4(3H)-one in the label approved on 07/23/2014 for ZYDELIG, FDA NDA no. 205858.

Idelalisib is a drug used for the treatment of chronic lymphocytic leukaemia. The substance acts as a phosphoinositide 3-kinase (PI3K) inhibitor. Specifically, it blocks P110δ, the delta isoform of PI3K.

WO 2005/113554 A2 is directed to the synthesis of idelalisib and to compounds structurally related to idelalisib. In the last step of the synthesis of idelalisib, the crude product is purified by column chromatography, dissolved in ethanol and concentrated in vacuo to obtain a mixed ethanol/water solvate of idelalisib.

WO 2013/134288 A1 describes seven polymorphic forms of idelalisib. Forms I and II are crystalline and anhydrous. Forms III to VII are solvates of iso-propyl alcohol (IPA), dimethylformamide (DMF), dimethylsulfoxide (DMSO), dichloromethane (DCM) and ethanol, respectively. Forms III and VII contain varying amounts of water. Additionally, it is known that Form I easily converts at least partially to Form II under pressure conditions which are typical for tablet preparation.

Idelalisib is commercially available under the trade name Zydelig®. However, Zydelig® tablets contain a mixture of both Forms I and II of idelalisib.

The solubility of Forms I and II of idelalisib is very low. At pH 6.8 a solubility of about 70 mg/L was measured.

It is therefore an object of the present invention to provide a solid form of idelalisib having an improved solubility compared to the polymorphic Form I and Form II described in WO 2013/134288. It is a further object of the invention to provide a solid form of idelalisib that allows the preparation of a pharmaceutical dosage form which is bioequivalent to the pharmaceutical composition approved with the US NDA no. 205858. It is a further object of the invention to provide a solid form of idelalisib that allows the preparation of a pharmaceutical dosage form wherein the solid state of the idelalisib is uniform. It is a further object of the invention to provide a solid form of idelalisib which is stable under compression and storage conditions. It is a further object of the invention to provide a solid form of idelalisib that allows the preparation of a pharmaceutical dosage having predictable and reproducible dissolution properties. It is a further object of the invention to provide a solid form of idelalisib that enables the provision of dosage forms having predictable and reproducible pharmacokinetic properties in patients.

It is further an object of the present invention to provide a process for the preparation of such a solid form of idelalisib.

### Summary of the Invention

According to the present invention, the above problem is unexpectedly overcome by amorphous idelalisib, especially by pure amorphous idelalisib. In particular it has been surprisingly found that pure and stable amorphous idelalisib can be produced using novel (crystalline) solvates of idelalisib as intermediates. Specifically, it has been unexpectedly found that tert-butanol, tetrahydrofuran (THF) and N,N-dimethylformamide (DMF) solvates of idelalisib are useful for the preparation of pure amorphous idelalisib on an industrial scale.

Amorphous idelalisib produced using these novel solvates according to the present invention is essentially free from any crystalline forms of idelalisib, such as for example idelalisib crystalline Form I. Further, amorphous idelalisib according to the present invention is stable under tableting conditions as well as under high temperature/high humidity conditions and has improved solubility compared to crystalline forms of idelalisib known in the prior art. Further, it has been surprisingly found that amorphous idelalisib prepared from these new solvates is essentially free from chemical impurities. Preparing an amorphous compound essentially free from chemical impurities is often a challenging task. Yet further, the amorphous idelalisib according to the present invention has a very low content of residual solvents. Preparing an amorphous compound having a very low content of residual solvents is often a challenging task.

Thus, in a first aspect, the present invention relates to amorphous idelalisib having no noticeable peak in an X-ray powder diffractogram, when measured at a temperature of 25°C with Cu K-alpha radiation.

In a further aspect, the present invention is directed to a process for the preparation of amorphous idelalisib comprising the steps of:
(i) providing an idelalisib solvate,
(ii) dissolving the idelalisib solvate of step (i) in a solvent, and
(iii) removing the solvent.

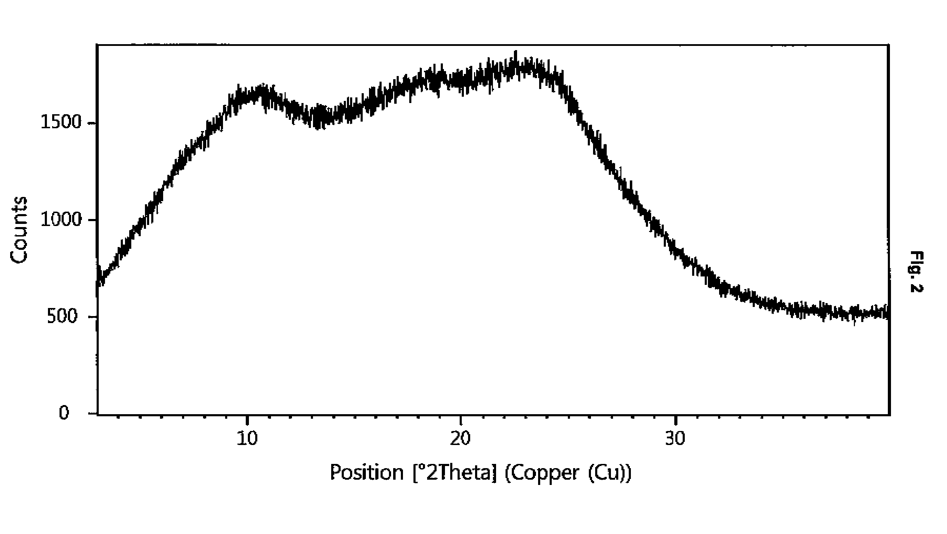

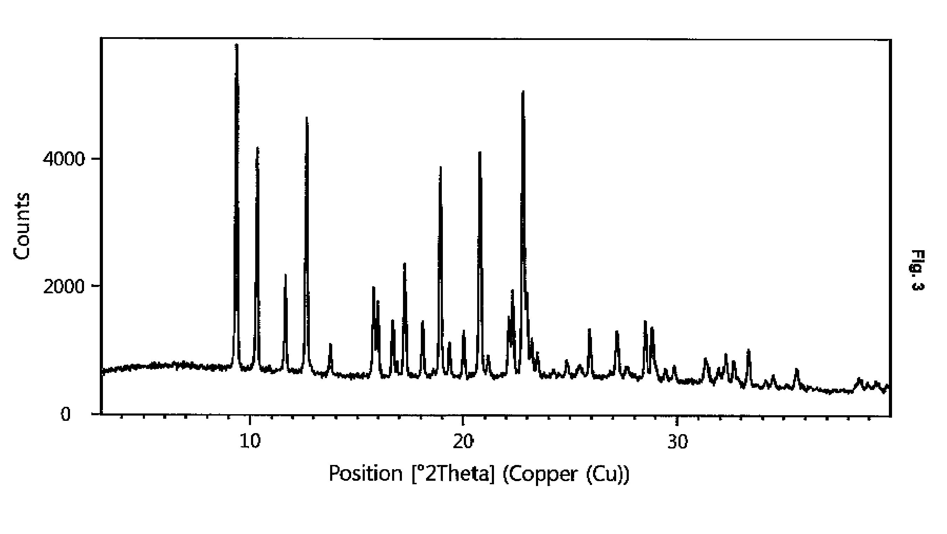

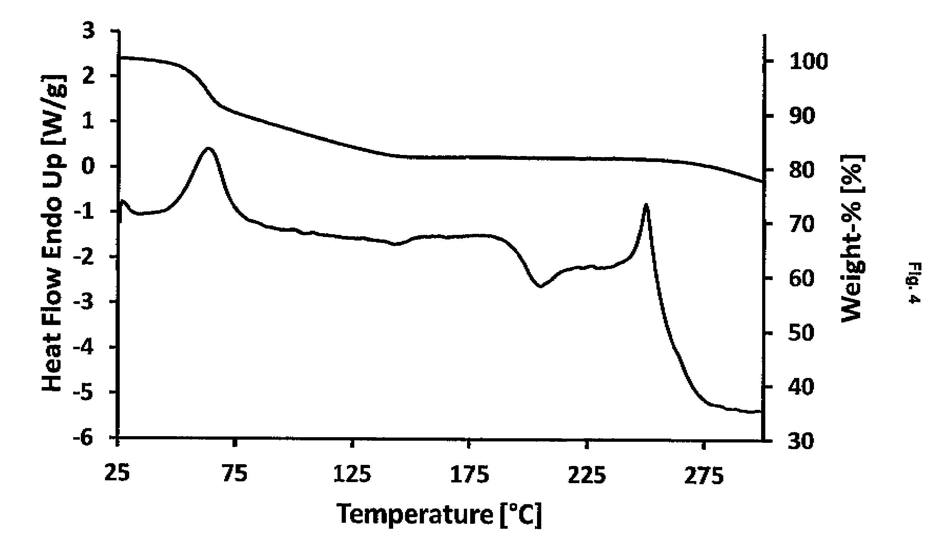

In a further aspect, the invention relates to crystalline idelalisib solvates selected from
A) tert-butanol solvate characterized by data selected from one or more of the following: an X-ray powder diffraction pattern with characteristic peaks at 9.4±0.2, 18.9±0.2, 22.8±0.2° 2-Theta when measured at a temperature of 25°C with Cu K-alpha radiation; an X-ray powder diffraction pattern substantially as depicted in Fig. 2; and by a combination of these data;
B) N,N-dimethylformamide solvate characterized by data selected from one or more of the following: an X-ray powder diffraction pattern with characteristic peaks at 11.8±0.2, 18.3±0.2, 20.1±0.2° 2-Theta when measured at a temperature of 25°C with Cu K-alpha radiation; an X-ray powder diffraction pattern substantially as depicted in Fig. 3; and by a combination of these data; and
C) tetrahydrofuran solvate characterized by data selected from one or more of the following: an X-ray powder diffraction pattern with characteristic peaks at 17.5±0.2, 19.4±0.2, 21.5±0.2° 2-Theta when measured at a temperature of 25°C with Cu K-alpha radiation; an X-ray powder diffraction pattern substantially as depicted in Fig. 4; and by a combination of these data.

In a further aspect, the present invention relates to a process for the preparation of idelalisib solvates of the present invention comprising the steps of
(I) providing a solution of idelalisib in the corresponding solvent,
(II) optionally adding seed crystals,
(III) stirring the solution, or performing one or more of temperature cycling, crash cooling, evaporation, or anti-solvent addition to form a slurry,
(IV) isolating the crystalline solvate in solid form, and
(V) optionally drying the crystalline solvate.

In a further aspect, the invention concerns a process for the purification of crude idelalisib, comprising the steps of:
(I) providing a solution of crude idelalisib in an organic solvent,
(II) optionally adding seed crystals, and
(III) stirring the solution, or performing one or more of temperature cycling, crash cooling, evaporation, or anti-solvent addition to form a slurry,
(IV) isolating the crystalline solvate in solid form,
(V) dissolving the crystalline solvate of idelalisib of step (IV) in a solvent,
(VI) removing the solvent, and
(VII) optionally drying the product obtained in step (VI),
in order to yield amorphous, preferably pure amorphous idelalisib.

In a further aspect, the invention relates to the use of a crystalline solvate of idelalisib for the preparation of amorphous idelalisib of the present invention.

In a further aspect, the invention relates to amorphous idelalisib as described herein or to a crystalline solvate of idelalisib as described herein for use in the treatment of cancer.

In a further aspect, the invention relates to a pharmaceutical composition comprising amorphous idelalisib as described herein or a crystalline solvate of idelalisib as described herein, and one or more pharmaceutically acceptable excipients.

### Brief Description of the Drawings

Fig. 1 shows an X-ray powder diffraction (XRPD) pattern of amorphous idelalisib prepared by precipitation from tert-butanol solvate of idelalisib.
Fig. 2 shows an X-ray powder diffraction (XRPD) pattern of amorphous idelalisib after compression at 740 MPa for 4.5 days.
Fig. 3 shows an X-ray powder diffraction (XRPD) pattern of the tert-butanol solvate of idelalisib.
Fig. 4 shows a TG/DTA thermogram of the tert-butanol solvate of idelalisib.
Fig. 5 shows an X-ray powder diffraction (XRPD) pattern of the tetrahydrofuran solvate of idelalisib.
Fig. 6 shows a TG/DTA thermogram of the tetrahydrofuran solvate of idelalisib.
Fig. 7 shows an X-ray powder diffraction (XRPD) pattern of the N,N-dimethylformamide solvate of idelalisib.
Fig. 8 shows a TG/DTA thermogram of the N,N-dimethylformamide solvate of idelalisib.

### Detailed Description of the Invention

### Amorphous idelalisib

In a first aspect, the invention is directed to amorphous idelalisib. Amorphous idelalisib in the context of the present invention means isolated amorphous idelalisib which is essentially free from any crystalline form of idelalisib, such as Form I or Form II. Further, preferably the amorphous idelalisib according to the present invention is essentially pure.

"Essentially free" in the context of the present invention means that no crystalline form, such as idelalisib crystalline Form I, can be detected by X-ray powder diffraction (XRPD) measurement, i.e. no peaks of a crystalline form of idelalisib can be observed in an XRPD measurement. Consequently, the amorphous idelalisib of the present invention is preferably phase pure. While a crystalline phase usually produces a distinctive XRPD pattern comprising sharp lines/peaks, amorphous materials produce a broad background signal. Hence, in an embodiment of the invention the amorphous idelalisib of the present invention preferably has a phase purity of 90% or more, more preferably of 95% or more, most preferably of 98% or more, as determined via XRPD.

In a further aspect, the invention is directed to amorphous form of idelalisib having no noticeable peak in a powder X-ray diffraction. "No noticeable peak" in the context of the present invention means that the XRPD does not contain any peak that the person skilled in the art would recognize as being characteristic of a crystalline phase. A peak characteristic of a crystalline phase is generally a sharp line. Hence, a peak characteristic of a crystalline phase is regarded as a peak having a width at half of its height from the top to its root, i.e. where the peak starts from the baseline, of less than 1° 2-Theta. Further, a peak characteristic of a crystalline phase is regarded as a peak having a height to width ratio of 5 : 1 or more, wherein the width is measured at half of its height. Additionally, a peak characteristic of a crystalline phase cannot be confused by the person skilled in the art with a peak of the background, since a peak characteristic of a crystalline phase has an at least 5 times greater height than the height of the highest peaks of which the background line consists, preferably at least 10 times greater.

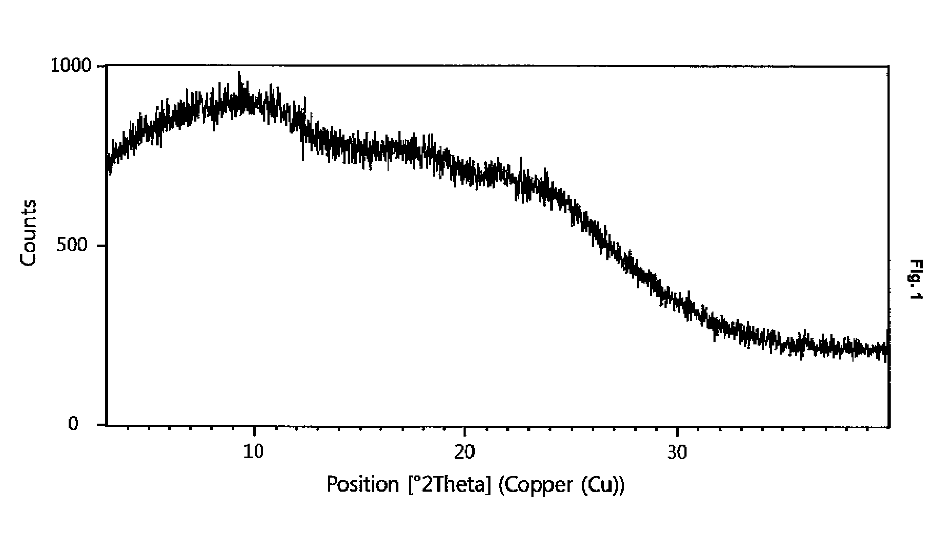

In a further embodiment, the amorphous idelalisib has an XRPD pattern substantially as shown in Fig. 1. "Substantially as shown in" in the context of the present invention means a pattern that is not necessarily identical to those depicted herein, but falls within the limits of experimental or statistical error or deviations when considered by the person skilled in the art. In view of the XRPD pattern of amorphous idelalisib, "substantially as shown in" means the absence of peaks characteristic of a crystalline phase, while the background line or curve may have a different aspect. For example, the amorphous idelalisib having an XRPD pattern as shown in Fig. 2 has an XRPD pattern substantially as shown in Fig. 1, since in Fig. 2 no noticeable peaks are present.

Amorphous idelalisib of the present invention is preferably pure in view of impurities derived from compounds other than idelalisib. The terms "pure" or "substantially pure" in the context of the present invention refer to chemical purity of the amorphous idelalisib. Chemical purity of the amorphous idelalisib can for example be determined by HPLC analysis. The amorphous idelalisib of the present invention has a chemical purity of 95 wt.% or more, preferably of 98 wt.% or more, more preferably of 99.5 wt.% or more, based on the total weight of the amorphous idelalisib.

In particular, amorphous idelalisib can be prepared according to the method of the present invention without using column chromatography. As a consequence, any possible product contamination with silica can be avoided. In an embodiment of the invention, the amorphous idelalisib thus preferably has a content of silica which is less than 0.5 wt.%, preferably is less than 0.3 wt.%, more preferably is less than 0.1 wt.%, further more preferably is less than 0.03 wt.%, most preferably is less than 0.01 wt%, based on the total weight of the amorphous idelalisib, which can be determined by sulphated ash analysis according to Ph. Eur. 6.0, 2.4.14.

It is a further advantage that amorphous idelalisib obtained in the present invention has a decreased level of residual solvents compared to the idelalisib solid forms known in the art.

The types and amounts of residual solvent present in amorphous idelalisib can be determined by gas chromatography as described in detail in chapter 467 of USP 37 NF32 (The United States Pharmacopeia, as official from December 1, 2014 to April 30, 2015) by following the sequence of procedure A, procedure B and procedure C as explained in detail on pages 219 to 223, which are hereby incorporated by reference.

The cumulative amounts of class 1 and class 2 solvents in the obtained amorphous idelalisib thus analyzed was detected to be less than 300 ppm, preferably less than 100 ppm, more preferably less than 50 ppm. Amorphous idelalisib of the invention is essentially free of THF. Residual ICH class 2 solvents are not detectable.

The International Conference on Harmonisation of Technical Requirements for Registration of Pharmaceuticals for Human Use (ICH) has divided common solvents in classes reflecting their toxicity (cf. for example Ph. Eur. 6.0, chapter 5.4). Additionally, the ICH makes recommendations as to what amounts of residual solvents are considered safe in pharmaceuticals.

Solvents in Class 1 should not be employed in the manufacture of drug substances, excipients, and drug products because of their unacceptable toxicity or their deleterious environmental effect. However, if their use is unavoidable in order to produce a drug product with a significant therapeutic advance, then their levels should be restricted. Solvents in Class 1 comprise for example benzene, carbon tetrachloride, 1,2-dichloroethane, 1,1-dichloroethene and 1,1,1-trichloroethane.

Solvents in Class 2 should be limited in pharmaceutical products because of their inherent toxicity. Permitted daily exposures (PDEs) are given to the nearest 0.1 mg/day, and concentrations are given to the nearest 10 ppm.

Solvents in Class 3 may be regarded as less toxic and of lower risk to human health. Class 3 includes no solvent known as a human health hazard at levels normally accepted in pharmaceuticals. It is considered that amounts of these residual solvents of 50 mg per day or less (corresponding to 5,000 ppm) would be acceptable without justification.

Finally, Class 4 solvents may also be of interest to manufacturers of excipients, drug substances, or drug products. However, no adequate toxicological data on which to base a PDE were found. ICH Class 4 solvents comprise isooctane, isopropyl ether, petroleum ether and trichloroacetic acid.

More details can be obtained by referring USP 37 Chapter <467> Residual Solvents.

Thus, in an embodiment, the present invention relates to amorphous idelalisib wherein at most 300 ppm of ICH Class 2 solvents are present. Preferably, at most 200 ppm, such as at most 150 ppm, such as at most 100 ppm, such as at most 90 ppm, at most 80 ppm, at most 70 ppm, at most 60 ppm, at most 50 ppm, at most 40 ppm, at most 30 ppm, at most 20 ppm and most preferably at most 10 ppm residual ICH Class 2 solvents are present. The present invention also relates to amorphous idelalisib which is substantially free of ICH Class 2 solvents, in particular essentially free from tetrahydrofuran and N,N-dimethylformamide. Preferably, the obtained amorphous idelalisib of the present invention has an overall organic volatile impurity content of up to 300 ppm, such as up to 100 ppm, for example 50 ppm, and being essentially free from ICH Class 2 solvents, such as tetrahydrofuran and N,N-dimethylformamide.

It is a further advantage of the amorphous idelalisib of the present invention that it is stable at high temperatures and/or high relative humidity.

In an embodiment of the invention, the amorphous idelalisib does not have any noticeable peak, especially any peak characteristic of a crystalline form of idelalisib, in a powder X-ray diffraction after 120 hours storage at 120°C. Preferably, the amorphous idelalisib has an XRPD pattern substantially as shown in Fig. 1 after 120 hours storage at 120°C.

In a further embodiment of the invention, the amorphous idelalisib does not have any noticeable peak, especially any peak characteristic of a crystalline form of idelalisib, in a powder X-ray diffraction after 120 hours storage at 40°C/75% relative humidity. Preferably, the amorphous idelalisib has an XRPD pattern substantially as shown in Fig. 1 after 120 hours storage at 40°C/75% relative humidity.

In yet a further embodiment of the invention, the amorphous idelalisib does not have any noticeable peak, especially any peak characteristic of a crystalline form of idelalisib, in a powder X-ray diffraction after 120 hours storage at 25°C/98% relative humidity. Preferably, the amorphous idelalisib has an XRPD pattern substantially as shown in Fig. 1 after 120 hours storage at 25°C/98% relative humidity.

The pure amorphous idelalisib of the present invention may be further characterized by phase stability under tableting conditions. While the anhydrous crystalline Form I converts at least partially to a crystalline Form II under pressure as applied during tableting, leading to a mixture of Forms I and II, such as in the commercially available product Zydelig®, the amorphous idelalisib of the present invention does preferably not show any substantial phase conversion under compression.

Thus, in one embodiment of the invention the amorphous idelalisib has an XRPD pattern substantially as shown in Fig. 2 after compression at 740 MPa for 4.5 days. More specifically, an XRPD pattern of amorphous idelalisib prior to compression is substantially as shown in Fig. 1 while an XRPD pattern of amorphous idelalisib after compression is substantially as shown in Fig. 2. The XRPD patterns illustrated in Fig. 1 and 2 are regarded as being identical within statistical error.

### Preparation of amorphous idelalisib

The invention further relates to a process for the formation of pure amorphous idelalisib as described above. In particular, it has been surprisingly found in the present invention that pure amorphous idelalisib can be prepared by using solvates of idelalisib, preferably by using the solvates of the present invention which are described below. After removing the solvent from the solvates of idelalisib, amorphous idelalisib with higher purity than the starting material can be obtained. Thus, it is possible with the process of the present invention to obtain 1) amorphous idelalisib and 2) with a purity which could not be obtained with the conventional processes described in the prior art.

In particular, the process for the preparation of amorphous idelalisib comprises the steps of:
(i) providing a solvate of idelalisib,
(ii) dissolving the solvate of idelalisib of step (i) in a solvent, and
(iii) removing the solvent.

Step (i) is not limited to specific solvates of idelalisib. Preferably, the solvates of idelalisib are solvates selected from tert-butanol, tetrahydrofuran, N,N-dimethylformamide, ethanol, dimethylsulfoxide (DMSO), isopropyl alcohol and dichloromethane. More preferably, the solvates of idelalisib are solvates selected from tert-butanol, tetrahydrofuran and N,N-dimethylformamide. Most preferably, the solvates of idelalisib are the new solvates of the present invention.

Preferably, step (ii) of dissolving the solvate of idelalisib of step (i) in a solvent is carried out at a temperature in the range of 20 to 50°C, more preferably in the range of 30 to 45°C.

Preferably, the concentration of the solvate of idelalisib in the solution obtained in step (ii) is in the range of 15 to 1000 mg/mL, more preferably in the range of 50 to 750 mg/mL. Most preferably, the solution of the solvate of idelalisib obtained in step (ii) is saturated.

Further, removing the solvent in step (iii) is typically performed by one or more of precipitation and filtration, thermal desolvation, hot melt-extrusion, spray drying, or lyophilization. Preferably, removing the solvent in step (iii) is performed by one or more of precipitation and filtration, spray drying, or lyophilization.

In one embodiment of the invention, removing the solvent in step (iii) is carried out by adding the solution of step (ii) into water and filtrating the precipitate. Preferably, the solvent in step (ii) is selected from ethanol, methanol, dioxane, tetrahydrofuran and N,N-dimethylformamide, as well as mixtures thereof. More preferably, the solvent in step (ii) is ethanol.

Preferably, the ratio between the volume of the solution of step (ii) and the volume of water to which the solution of step (ii) is added in order to lead to amorphous idelalisib to precipitate is in the range of 1:1 to 1:40, such as 1:2, 1:3, 1:4, 1:5, 1:6, 1:7, 1:8, 1:9, 1: 10, 1:15, 1:20, 1:30 and 1:35.

The filtration of the amorphous idelalisib is not limited to specific methods known in the art. The filtration of the amorphous idelalisib may therefore be carried out for example by centrifuge filtration.

Moreover, the product obtained in step (iii) may further optionally be dried, for example under reduced pressure, typically at room temperature, or heated up to a temperature between 25°C and 90°C.

In a further embodiment, removing the solvent in step (iii) is carried out by spray or freeze drying the solution of step (ii). Solvents in pure form or mixtures used for spray drying are preferably selected from Biopharmaceutics Classification System (BCS) Class 3 solvent(s), further preferably with a low boiling point, most preferably acetone and dichloromethane. Solvents used for lyophilization are typically selected from dioxane, dimethylsulfoxide (DMSO), tetrahydrofuran, acetic acid, acetone, ethanol, methanol and tert-butanol. Preferably, the solvent in step (ii) when step (iii) comprises a freeze drying (lyophilization) step is selected from dioxane, tetrahydrofuran, ethanol and methanol, as well as mixtures thereof. More preferably, the solvent in step (ii) is selected from dioxane, tetrahydrofuran and mixtures thereof.

The amount of the solvent is not particularly restricted, preferably a 10 to 100-fold amount with respect to the substrate. The time for lyophilization is not particularly restricted, preferably 1 to 240 hours, more preferably 2 to 120 hours.

### Solvates of Idelalisib

According to a further aspect, the present invention refers to novel crystalline solvates of idelalisib. As used herein, solvate of idelalisib is preferably synonymous to crystalline solvate of idelalisib, i.e. the solvates of idelalisib of the present invention are preferably isolated in solid form. The novel crystalline solvates can be used as intermediates in the preparation of pure amorphous idelalisib as described herein.

In an aspect, the present invention relates to a crystalline idelalisib solvate selected from a tert-butanol solvate, a tetrahydrofuran solvate and a N,N-dimethylformamide solvate.

Preferably, crystalline idelalisib tert-butanol solvate of the present invention is characterized by data selected from one or more of the following:
a) an X-ray powder diffraction pattern with characteristic peaks at 9.4±0.2, 18.9±0.2, 22.8±0.2° 2-Theta when measured at a temperature of 25°C with Cu K-alpha radiation;
b) an X-ray powder diffraction pattern substantially as depicted in Fig. 3;
and by a combination of these data.

Additionally, crystalline idelalisib tert-butanol solvate is preferably characterized by a thermogravimetric/differential thermal analysis (TG/DTA) thermogram substantially similar to the one set forth in Fig. 4, when measured at a rate of 10°C/min from 30°C to 300°C.

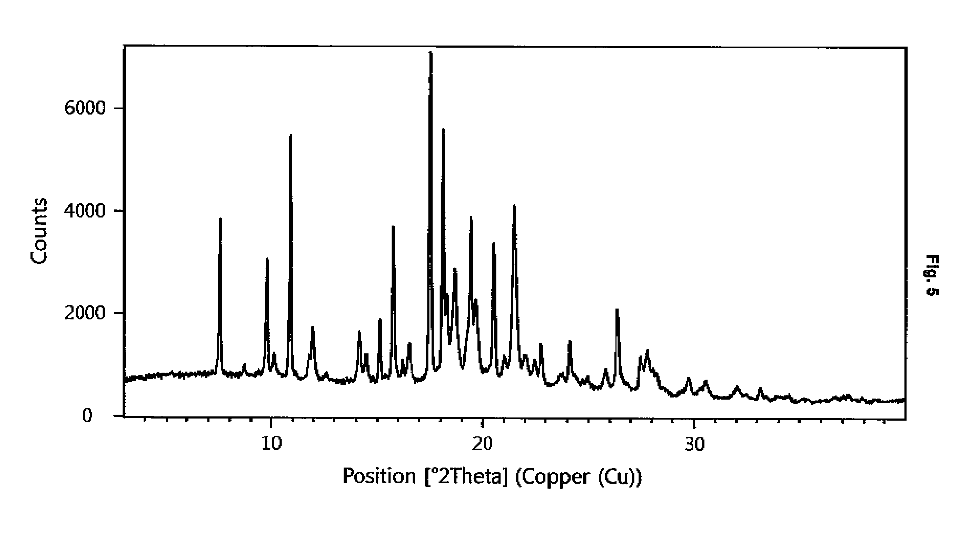

Preferably, crystalline idelalisib tetrahydrofuran solvate is characterized by data selected from one or more of the following:
a) an X-ray powder diffraction pattern with characteristic peaks at 17.5±0.2, 19.4±0.2, 21.5±0.2° 2-Theta when measured at a temperature of 25°C with Cu K-alpha radiation;
b) an X-ray powder diffraction pattern substantially as depicted in Fig. 5;
and by a combination of these data.

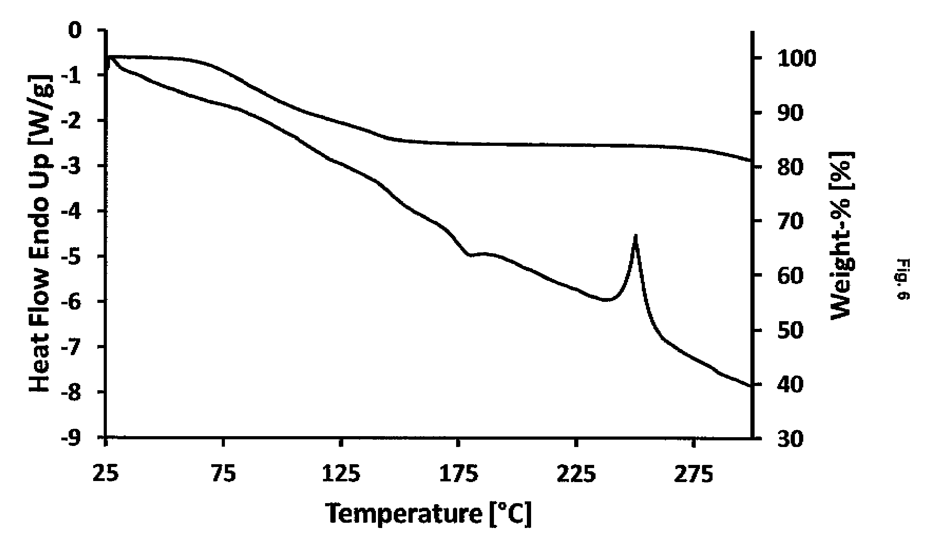

Additionally, crystalline idelalisib tetrahydrofuran solvate is preferably characterized by a thermogravimetric/differential thermal analysis (TG/DTA) thermogram substantially similar to the one set forth in Fig. 6, when measured at a rate of 10°C/min from 30°C to 300°C.

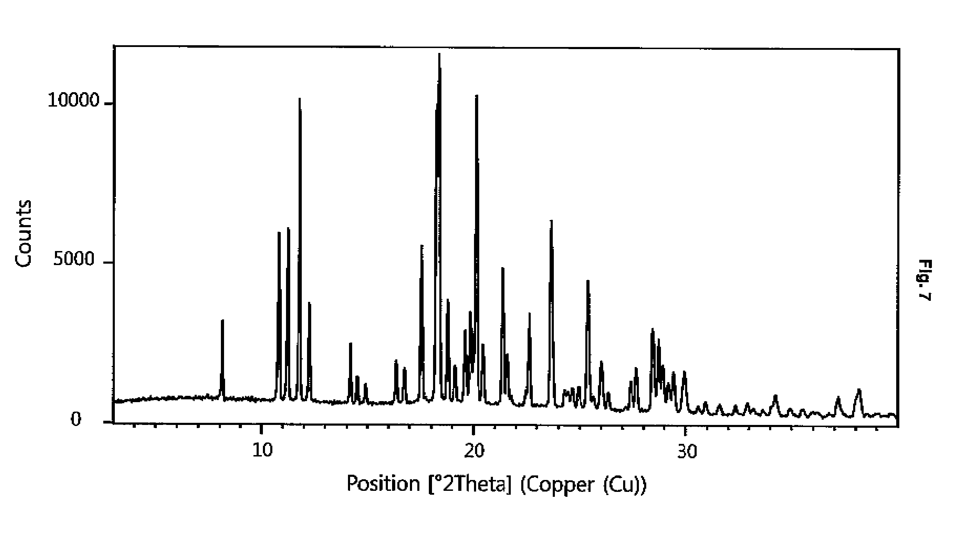

Preferably, crystalline idelalisib N,N-dimethylformamide solvate is characterized by data selected from one or more of the following:
a) an X-ray powder diffraction pattern with characteristic peaks at 11.8±0.2, 18.3±0.2, 20.1±0.2° 2-Theta when measured at a temperature of 25°C with Cu K-alpha radiation;
b) an X-ray powder diffraction pattern substantially as depicted in Fig. 7;
and by a combination of these data.

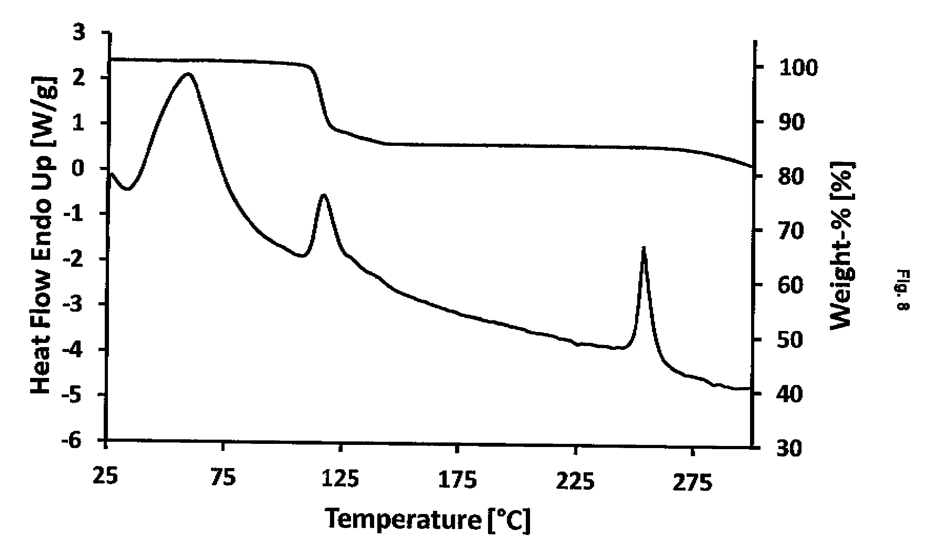

Additionally, crystalline idelalisib N,N-dimethylformamide solvate is preferably characterized by a thermogravimetric/differential thermal analysis (TG/DTA) thermogram substantially similar to the one set forth in Fig. 8, when measured at a rate of 10°C/min from 30°C to 300°C.

In particular, the crystalline solvates of idelalisib of the present invention may be characterized by the peaks listed in the following tables, showing the main 6 peaks (Table 1) and the main 10 peaks (Table 2) in an XRPD diffractogram.

**Table 1 - Peak selection (6 Peaks)**

| *tert*-butanol solvate | | THF solvate | | DMF solvate | |
|---|---|---|---|---|---|
| °2Theta (±0.2) | Rel. Int. [%] | °2Theta (±0.2) | Rel. Int. [%] | °2Theta (±0.2) | Rel. Int. [%] |
| 9.4 | 83 | 7.5 | 33 | 10.8 | 41 |
| 10.3 | 62 | 10.9 | 53 | 11.8 | 75 |
| 12.6 | 77 | 17.5 | 89 | 18.2 | 91 |
| 18.9 | 82 | 18.7 | 59 | 18.3 | 95 |
| 20.7 | 79 | 19.4 | 73 | 20.1 | 100 |
| 22.8 | 100 | 21.5 | 100 | 25.4 | 51 |

**Table 2 - Peak selection (10 Peaks)**

| *tert*-butanol solvate | | THF solvate | | DMF solvate | |
|---|---|---|---|---|---|
| °2Theta (±0.2) | Rel. Int. [%] | °2Theta (±0.2) | Rel. Int. [%] | °2Theta (±0.2) | Rel. Int. [%] |
| 9.4 | 83 | 7.5 | 33 | 10.8 | 41 |
| 10.3 | 62 | 9.8 | 31 | 11.8 | 75 |
| 12.6 | 77 | 10.9 | 53 | 17.5 | 47 |
| 15.8 | 32 | 17.5 | 89 | 18.2 | 91 |
| 17.2 | 36 | 18.1 | 66 | 18.3 | 95 |
| 18.9 | 82 | 18.7 | 59 | 20.1 | 100 |
| 20.7 | 79 | 19.4 | 73 | 21.3 | 44 |
| 22.8 | 100 | 21.5 | 100 | 23.6 | 72 |
| 28.8 | 26 | 26.4 | 34 | 25.4 | 51 |
| 38.6 | 25 | 28.1 | 26 | 28.4 | 33 |

In another preferred embodiment, the solvates of idelalisib of the present invention may be characterized by a stoichiometric composition of the solvates, i.e. the number of solvent molecules per idelalisib molecule. The stoichiometry can be determined by DTA/TG and/or 1H NMR.

In an embodiment, the idelalisib tert-butanol solvate preferably comprises tert-butanol in the range of 0.2 mol to 1.5 mol, more preferably in the range of 0.3 mol to 1.3 mol, most preferably in the range of 0.4 mol to 1.1 mol per mol idelalisib.

Preferably, the idelalisib tert-butanol solvate further comprises water. The tert-butanol solvate may contain water in the range of 0.5 to 5 mol per mol idelalisib, preferably in the range of 1 to 4 mol, more preferably in the range of 1.5 to 3.5 mol water per mol idelalisib.

In one embodiment, the idelalisib tert-butanol solvate comprises tert-butanol preferably in the range of 0.2 mol to 1.5 mol, more preferably in the range of 0.3 mol to 1.3 mol, most preferably in the range of 0.4 mol to 1.1 mol per mol idelalisib and water preferably in the range of 0.5 to 5 mol per mol idelalisib, more preferably in the range of 1 to 4 mol, most preferably in the range of 1.5 to 3.5 mol water per mol idelalisib.

In a further embodiment, the idelalisib tetrahydrofuran solvate preferably comprises tetrahydrofuran in the range of 0.1 mol to 1.0 mol, more preferably in the range of 0.2 mol to 0.8 mol, most preferably in the range of 0.3 mol 0.7 mol per mol idelalisib.

In a further embodiment, the idelalisib N,N-dimethylformamide solvate preferably comprises N,N-dimethylformamide in the range of 0.5 mol to 1.5 mol, more preferably in the range of 0.7 mol to 1.3 mol, most preferably in the range of 0.8 mol to 1.2 mol per mol idelalisib.

The idelalisib tetrahydrofuran and the N,N-dimethylformamide solvates may additionally comprise water in the range of 0 mol to 0.5 mol per mol idelalisib.

### Preparation of the Solvates of Idelalisib

The solvates of the present invention can be prepared by a process comprising the steps of
(I) providing a solution of idelalisib in the corresponding solvent,
(II) optionally adding seed crystals,
(III) stirring the solution, or performing one or more of temperature cycling, crash cooling, evaporation, or anti-solvent addition to form a slurry,
(IV) isolating the crystalline solvate in solid form, and
(V) optionally drying the crystalline solvate.

The organic solvent in step (I) is preferably selected from tert-butanol, tert-butanol/water, tetrahydrofuran and N,N-dimethylformamide.

Preferably, tert-butanol/water is a mixture of tert-butanol and water in a ratio in the range of 80:20 to 99.5:0.5, more preferably 85:15 to 99:1, most preferably 90:10 to 98:2 (v/v).

Dissolution of idelalisib in step (I) can be obtained at temperatures in the range of 4°C to 80°C depending on the solvent used, preferably in the range of 10 to 70 °C, more preferably in the range of 20 to 60°C, most preferably in the range of 30 to 50°C.

A crystalline form of idelalisib, such as Form I, Form II or a mixture of Forms I and II, or amorphous idelalisib can be used as the idelalisib to be dissolved in step (I).

Optionally, seed crystals may be added in step (II). The seed crystals are prepared by the same solvent used in step (I) of the process. In particular, the seed crystals are prepared by the same process steps as the solvates of the present invention.

The seed crystals are typically added in an amount of 0.1 wt% to 10 wt%, preferably in an amount of 0.5 wt% to 7.0 wt%, most preferably 1.0 wt.% 5.0 wt%, on the basis of the total amount of the starting material, i.e. the solution obtained in step (I).

In step (III) the solution is transformed into a slurry by performing one or more of stirring the solution, or submitting the solution to one or more of temperature cycling, crash cooling, evaporation, or anti-solvent addition.

Stirring is performed at a temperature of 10°C to 100°C, preferably between 20°C and 50°C, most preferably at about room temperature, e.g. 22 to 25°C, typically for a period of time of 2 h to 21 days.

Temperature cycling is preferably performed at atmospheric pressure, preferably at 2 to 22°C or 20 to 80°C, but also other conditions may be used depending on the type of solvate to be produced. Temperature cycling is typically performed for a period of 16 to 24 hours by subjecting the solution or slurry to temperature cycles such as between 20°C and 80°C, wherein each cycle may have a length of between 0.5 and 8 hours, preferably about 2 hours. A typical but not limiting temperature cycling protocol may be as follows:
i) Heat from 20°C to 80°C (1 h)
ii) Cool to 20°C (1 h).

Steps i) and ii) are typically repeated 5 to 10 times.

Crash cooling is typically performed by directly and rapidly cooling the solution from a temperature of between 25 and 50°C to a temperature of 2°C or below, such as a temperature between 0°C and -18°C, depending on the type of solvate, and keeping the solution at this temperature for example for 4 h to 24 h, such as for example 16 to 20 h.

Evaporation is typically performed at reduced pressure, but may also be achieved at atmospheric pressure.

The solvent used for anti-solvent addition may typically be selected from water, acetonitrile, a C1-4 dialkylether, preferably methyltertbutylether (MTBE), or an alkane, preferably is selected from an alkane, more preferably a C5 alkane, a C6 alkane, a C7 alkane, a C8 alkane, or a mixture of two or more thereof, more preferably a C7 alkane, more preferably n-heptane. Preferably, the anti-solvent is water or n-heptane.

The addition of anti-solvent may be carried out at a temperature in the range of 0 to 25°C.

Additional solvent may be added in step (III) in case of formation of thick slurries to allow easier stirring of the resulting slurry.

In an embodiment, step (III) comprises anti-solvent addition and evaporation. Specifically, the evaporation carried out after addition of anti-solvent leads to oily solutions or to clear solutions without precipitation. Optionally, the solutions may be sonicated before evaporation of the solvent.

Optionally, isolation in step (IV) may be performed by using procedures known in the art, such as by filtration, centrifugation, or evaporation of solvent.

Moreover, the isolated crystals may optionally be dried in step (V), e.g. under reduced pressure, typically at room temperature (25°C), or heated up to a temperature of between 25°C and 50°C or the crystals may directly be used in further processes, such as the preparation of amorphous idelalisib or isolated crystals may be used as seed crystals for the preparation of a solvate of idelalisib.

The invention further relates to the use of idelalisib solvates for the manufacture of amorphous idelalisib which can be obtained by the process of the invention in pure amorphous form. In particular, it has surprisingly been found in the present invention that amorphous idelalisib can be prepared by using the solvates of the present invention. After dissolving the solvates of the present invention in a solvent and removing the solvent, pure amorphous idelalisib can be obtained.

### Purification of Crude Idelalisib

In another embodiment, the present invention refers to a combination of the process for the preparation of the solvates of idelalisib and the process for the preparation of pure amorphous idelalisib, both as described herein. As crude idelalisib may be used as the starting material for the preparation of the solvates of idelalisib of the present invention, which may contain any chemical impurities, such as residual amounts of silica, or may contain residual amounts of crystal forms of idelalisib, such as the crystal form I of idelalisib, it is possible with the combination of both processes to obtain amorphous idelalisib with a higher purity than the starting material. Thus, the combination of both processes may be used as a process for purification of idelalisib, such as crude idelalisib. Crude idelalisib may be for example the mixed ethanol/water solvate of idelalisib obtained after column chromatography described in WO 2005/113554 A2.

"Crude" in the context of the present invention means having a chemical purity of less than 95 wt.%, based on the total weight of the idelalisib. Preferably, "crude" means having a phase purity of less than 90% in addition to the 95wt.% chemical impurity as described above. Phase purity of less than 90% comprises an amorphous form and a crystalline form having a phase purity of less than 90%, respectively.

Thus, in another aspect, the present invention refers to a process for the purification of crude idelalisib, comprising the steps of:
(I) providing a solution of crude idelalisib in an organic solvent,
(II) optionally adding seed crystals, and
(III) stirring the solution, or performing one or more of temperature cycling, crash cooling, evaporation, or anti-solvent addition to form a slurry,
(IV) isolating the crystalline solvate in solid form,
(V) dissolving the crystalline solvate of idelalisib of step (IV) in a solvent,
(VI) removing the solvent, and
(VII) optionally drying the product obtained in step (VI),

in order to yield amorphous, preferably pure amorphous idelalisib.

Amorphous idelalisib with a higher purity, i.e. chemical and phase purity, than the crude idelalisib used as the starting material in step (I) is obtained in step (VI) or optionally in step (VII). In particular, the amorphous idelalisib obtained by the above process is substantially free from silica or from residual amounts of crystal forms of idelalisib such as crystal Form I of idelalisib, which, however, may be present in the crude idelalisib used as the starting material in step (I) of the process.

The preferred embodiments of the process are the same as described above for the process for the preparation of the solvates of idelalisib of the present invention and the process for the preparation of pure amorphous idelalisib and thus also refer to the combination of both processes.

### Pharmaceutical Composition

In a further aspect of the invention, the amorphous idelalisib of the present invention or a crystalline solvate of idelalisib as described herein is used in the treatment of cancer. In particular, cancer may be hematologic malignancy, preferably a B cell malignancy, preferably selected from chronic lymphocytic leukemia (CLL), small lymphocytic lymphoma (SLL), mantle cell lymphoma (MCL), non-Hodgkin's lymphoma, indolent non-Hodgkin's lymphoma, diffuse large B Cell lymphoma (DLBCL), multiple myeloma (MM), marginal zone lymphoma (NHL), hairy cell leukemia, acute lymphocyte leukemia (ALL), acute myeloid leukemia (AML).

Consequently, the present invention also refers to a pharmaceutical composition comprising amorphous idelalisib as described herein or a crystalline solvate of idelalisib as described herein, as an active pharmaceutical ingredient.

The present invention also relates to a pharmaceutical composition comprising amorphous idelalisib as an active pharmaceutical ingredient, and wherein at most 300 ppm of ICH Class 2 solvents are present. Preferably, at most 200 ppm, such as at most 150 ppm, such as at most 100 ppm, such as at most 90 ppm, at most 80 ppm, at most 70 ppm, at most 60 ppm, at most 50 ppm, at most 40 ppm, at most 30 ppm, at most 20 ppm and most preferably at most 10 ppm residual ICH Class 2 solvents are present in the pharmaceutical composition of the invention. The present invention also relates to a pharmaceutical composition comprising amorphous idelalisib which is substantially free of ICH Class 2 solvents, in particular essentially free from tetrahydrofuran and N,N-dimethylformamide.

In an embodiment, the pharmaceutical composition of the present invention contains as active pharmaceutical ingredient only amorphous idelalisib of the present invention.

In a further embodiment, the pharmaceutical composition of the present invention comprises idelalisib, wherein at least 90 wt.%, such as 95 wt.%, is amorphous idelalisib according to the present invention, based on the weight of idelalisib present in the pharmaceutical composition.

Preferably, the pharmaceutical composition further comprises one or more pharmaceutically acceptable excipients. Preferably, the one or more pharmaceutically acceptable excipients are selected from binders, fillers, lubricants, glidants, disintegrants, sweeteners, antioxidants, preservatives and colorants, more details of these excipients described in subsequent embodiments.

The pharmaceutical composition is typically a solid oral dosage form. It may be administered to a subject by multiple administration routes, including but not limited to, oral (non-limiting examples for suitable dosage forms include solid dosage forms, like tablets, powders, capsules, suppositories, sachets, troches and lozenges), parenteral (( non-limiting examples. intravenous, subcutaneous, intramuscular), intranasal, buccal, topical, rectal, or transdermal administration routes. More details of oral solid dosage form compositions described in subsequent embodiments.

In an embodiment of the invention, the pharmaceutical composition is a compressed solid oral dosage form. In an embodiment of the invention, the compressed dosage form is essentially free of crystalline idelalisib.

In one aspect, the present invention features a pharmaceutical composition comprising a stabilized pharmaceutical composition, wherein the composition comprises
(1) Idelalisib in an amorphous form,
(2) a pharmaceutically acceptable organic or inorganic matrix, and
(3) a pharmaceutically acceptable surfactant

In certain embodiments, of the invention a particular ratio of API idelalisib: matrix by weight is from 1 :0.25 to 1: 10.

Suitable ratios are 1:2, 1:3 and 1:4.

In certain embodiments, one or more surfactants will be present in the stabilized pharmaceutical composition in an amount of 0.1 to 50%, preferably :0.5% (eg, 1 to 2%) by weight of the solid dispersion. The presence of a surfactants provides a further enhancement of the increase in therapeutic potential achieved with the present invention.

In one embodiment of this aspect of the invention, the organic matrix is selected from homopolymer of N-vinyl lactam, copolymer of N-vinyl lactam, cellulose ester, cellulose ether, polyalkylene oxide, polyacrylate, polymethacrylate, polyacrylamide, polyvinyl alcohol, vinyl acetate polymer, oligosaccharide, or polysaccharide. Non-limiting examples of suitable hydrophilic polymers include homopolymer of N-vinyl pyrrolidone, copolymer of Nvinyl pyrrolidone, copolymer of N-vinyl pyrrolidone and vinyl acetate, copolymer of N-vinyl pyrrolidone and vinyl propionate, graft copolymer of polyethylene glycol/polyvinyl caprolactam/polyvinyl acetate (e.g., Soluplus), polyvinylpyrrolidone, methylcellulose, ethylcellulose, hydroxyalkylcelluloses, hydroxypropylcellulose, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose succinate, hydroxypropylmethylcellulose acetate succinate, polyethylene oxide, polypropylene oxide, copolymer of ethylene oxide and propylene oxide, methacrylic acid/ethyl acrylate copolymer, methacrylic acid/methyl methacrylate copolymer, butyl methacrylate/2-dimethylaminoethyl methacrylate copolymer, poly(hydroxyalkyl acrylate), poly(hydroxyalkyl methacrylate), copolymer of vinyl acetate and crotonic acid, partially hydrolyzed polyvinyl acetate, carrageenan, galactomannan, or xanthan gum, or a combination thereof. In some cases, carbon black, cellulose, cellulose derivatives, polyols, sugars, sugar alcohols and other sugar derivatives (e.g. , lactose, mannitol), starches, pre-gelatinized starches, starch derivatives, modified, starches, dextrins, maltodextrins, polydextroses, dextroses, or their mixtures can be used

Non-limiting examples of a preferred organic matrix for the invention include polyvinylpyrrolidone (PVP) K17, PVP K25, PVP K30, PVP K90, hydroxypropyl methylcellulose (HPMC) E3, HPMC E5, HPMC E6, HPMC E15, HPMC K3, HPMC A4, HPMC A15, HPMC acetate succinate (AS) LF, HPMC AS MF, HPMC AS HF, HPMC AS LG, HPMC AS MG, HPMC AS HG, HPMC phthalate (P) 50, HPMC P 55, Ethocel4, Ethocel 7, Ethocel10, Ethocel14, Ethocel20, copovidone (vinylpyrrolidone-vinyl acetate copolymer 60/40), polyvinyl acetate, methacrylate/methacrylic acid copolymer (Eudragit) L100-55, Eudragit L100, Eudragit S 100, polyethylene glycol (PEG) 400, PEG 600, PEG 1450, PEG 3350, PEG 4000, PEG 6000, PEG 8000, Soluplus, poloxamer 124, poloxamer 188, poloxamer 237, poloxamer 338, and poloxamer 407 or a combination thereof.

Non-limiting examples for a suitable inorganic matrix are Al2O3, MgO, TiO2 ZnO, calcium carbonate, calcium phosphate, calcium sulphate, Magnesium Alumino-metasilicates like Neusilin , dibasic calcium phosphates like Fujicalin, different grades of Melt-F or silicon dioxide, more preferably colloidal silicon dioxide or precipitated silicon dioxide characterized by high surface area due to open pores. Suitable carriers are for example, Aerosil® 90, 130,150, 200 or 380 or Aerosil® OX 50, EG 50 or TT 600 (Evonik Degussa GmbH, Germany), or Syloid series such as Syloid 244 or Syloid AL-1 (Grace Davison, USA), HDK pyrogenic silica series such as HDK N20 (Wacker Chemie AG, Germany) can be used. Preferably Aerosil® 200 or Syloid 244 can be used, more preferably Syloid AL-1 can be used.

Methods for the preparation of these organic and inorganic matrix stabilized formulations are known in the art by melting (e.g. hot-melt extrusion, spray congrealing and melt granulation) or by typically comprising the steps of dissolving the drug and the matrix in a common solvent and evaporating the solvent (e.g. spray drying, solvent casting or freeze drining) or by using other technologies (e.g. milling, ball milling solvent controlled precipitation, pH controlled precipitation, supercritical fluid technology and cryogenic co milling).

In certain embodiments the formulation product or dose may comprise in addition one or more fillers, binders, disintegrants, plasticisers and/or lubricants.

Typically the combined amount of excipients comprises, for example, 1 to 90% by weight of the formulation or dose.

In certain embodiments, the idelalisib will be present in an amount of 10 to 70%, and preferably from 15 to 50% (more preferably 20 to 30% or 25 to 35%) by weight of the solid dispersion.

In certain embodiments, one or more fillers will be present in an amount of 1 to 70% by weight of the formulation or dose. In certain embodiments, one or more binders will be present in an amount of 2 to 40% by weight of the formulation or dose.

In certain embodiments, one or more disintegrants will be present in an amount of 1 to 25 20%, and especially 4 to 10% by weight of the formulation or dose.

It will be appreciated that a particular excipient may act as both a binder and a filler, or as a binder, a filler and a disintegrant.

In certain embodiments, one or more lubricants will be present in an amount of 0.5 to 3%, and especially 1 to 2% by weight of the formulation or dose.

In certain embodiments, one or more plasticisers will be present in the solid dispersion in an amount of0.1% to 50%, preferably: 0.5% (e.g. 1 to 2%) by weight of the solid dispersion. The presence of a plasticiser may enhance processability of the solid dispersion, for example when a melt extrusion process is used.

Non-limiting examples for suitable fillers include, for example, starches and modified starches, such as maize starch, potato starch, rice starch, wheat starch,pregelatinized starch, fully pregelatinized starch; cellulose derivatives (e.g. microcrystalline cellulose, cellulose or silicified microcrystalline cellulose), sugars and sugar derivatives such as mannitol, erythritol; lactose, xylitol and lactitol such as lactosemonohydrate, lactose anhydrous, spray dried lactose or milled lactose; inorganic salts like calcium sulphate, calcium phosphate, calcium hydrogenphosphate, calcium carbonate

Non-limiting examples for suitable binders include, for example, lactose, starches, modified starches, sugars, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone, gelatine, polyvinylpyrollidone (PVP) and sodium alginate

Non-limiting examples for suitable disintegrants include, for example, carmellose calcium, carboxymethylstarch sodium, croscarmellose sodium (cellulose carboxymethylether sodium, salt, crosslinked), starch, such as sodium starch glycolate or corn starch, crosslinked polyvinylpyrrolidone (crospovidone), and low-substituted hydroxypropylcellulose, microcrystalline cellulose,hydroxypropyl and methylcellulose

Non-limiting examples for suitable lubricants include, for example, stearic acid, glyceryl behenate, magnesium stearate, magnesium lauryl stearate, sodium stearyl fumarate, stearic acid, calcium stearate, zinc stearate, potassium benzoate, sodium benzoate, myristic acid, palmitic acid, mineral oil, hydrogenated castor oil, medium-chain triglycerides, poloxamer, polyethylene glycol and talc.

Non-limiting examples of pharmaceutically acceptable surfactants that are suitable for the present invention:
Include non-limiting examples of suitable anionic surfactants such as sodium dodecyl sulphate (sodium lauryl sulphate); docusate sodium;
Include non-limiting examples of suitable cationic surfactants such as cetrimide, benzethonium chloride, cetylpyridinium chloride and lauric acid;
Include non-limiting examples of suitable nonionic surfactants such as polyoxyethylene castor oil derivates, e.g.polyoxyethyleneglycerol triricinoleate or polyoxyl 35 castor oil (Cremophor EL; BASF Corp.) or polyoxyethyleneglycerol oxystearate such as polyethylenglycol 40 hydrogenated castor oil (Cremophor RH 40, also known as polyoxyl 40 hydrogenated castor oil or macrogolglycerol hydroxystearate) or polyethylenglycol 60 hydrogenated castor oil (Cremophor RH 60); or a mono fatty acid ester of polyoxyethylene sorbitan, such as a mono fatty acid ester of polyoxyethylene (20) sorbitan, e.g. polyoxyethylene (20) sorbitan monooleate (Tween 80), polyoxyethylene (20) sorbitan monostearate (Tween 60), polyoxyethylene (20) sorbitan monopalmitate (Tween 40), or polyoxyethylene (20) sorbitan monolaurate (Tween 20). As described above, a mixture of surfactants can be used in a solid composition of the present invention Other suitable ionic or non-ionic surfactants may also be used.

Non-limiting examples of suitable plasticisers include: acetyltributyl citrate, acetyltriethyl citrate, benzyl benzoate, chlorbutanol, dextrin, dibutyl phthalate, diethyl phthalate, dimethyl phthalate, glycerine, glycerine monostearate, mannitol, mineral oil, lanolin alcohols, palmitic acid, polyethylene glycol, polyvinyl acetate phthalate,propylene glycol, 2-pyrrolidone, sorbitol, stearic acid, triacetin, tributyl citrate,triethanolamine and triethyl citrate, KolliporTMTPGS, KolliporTMRH40, KolliporTMP188, KolliporTMP407.

Additional excipients may be included in the formulation or dose.

Non-limiting additional conventional excipients, which may be added, include preservatives, stabilizers, anti-oxidants, silica flow conditioners, antiadherents or glidants, sweetening and other flavouring agents, colouring agents coating materials, preservatives, dyes, thickeners, adjuvants, antimicrobial agents and glidants, controlled release agents for the various formulation types.

Non-limiting examples of suitable glidants are selected from the group consisting of colloidal silica, hydrophobic colloidal silica and magnesium trisilicate, such as talcum.

Non-limiting examples of suitable controlled release (CR) agents for the production of matrix based CR solid dosage forms are preferably selected from the group consisting of high viscosity water soluble polymerssuch as hydroxypropyl cellulose and hypromellose, and lypophilic matrix forming agents such as glyceryl behenate;

Non-limiting examples of suitable sweeteners are selected from the group consisting of aspartame, saccharin sodium, dipotassium glycyrrhizinate, aspartame, stevia, thaumatin,

Non-limiting examples for suitable coating materials are gelatin, wax, shellac or biological degradable polymers. iron oxide

Non-limiting examples for suitable preservatives are methyl-, propyl, -or butylparabens, propylene paraben, sorbic acid, chlorobutanol, phenol, thimerosal, potassium sorbate, glycerin, propylene glycol, cysteine and/or methionine.

Non-limiting examples for suitable thickeners are synthetic polymers, fatty acids and fatty acid salts and esters and fatty alcohols.

Non-limiting other suitable fillers, binders, disintegrants, lubricants and additional excipients which may be used are described in the Handbook of Pharmaceutical Excipients, 5th Edition (2006);The Theory and Practice of industrial Pharmacy, 3rd Edition 1986; Pharmaceutical DosageForms 1998; Modem Pharmaceutics, 3rd Edition 1995; Remington's Pharmaceutical Sciences 20th Edition 2000.

In one embodiment, the formulation product is formed into a suitable dosage form ready for oral administration.

Capsules will typically contain the solid composition within a capsule which may be made of gelatin or other conventional encapsulating material.

Tablets and powders may be coated. Tablets and powders may be coated with an enteric coating.

The enteric coated powder forms may have coatings comprising phthalic acid cellulose acetate, hydroxypropylmethylcellulose phthalate, polyvinylalcohol phthalate, carboxymethylethylcellulose, a copolymer of styrene and maleic acid, a copolymer of methacrylic acid and methyl methacrylate, and like materials, and if desired, they may be employed with suitable plasticizers and/or extending agents. A coated tablet may have a coating on the surface of the tablet or may be a tablet comprising a powder or granules with an enteric coating.

Modified release formulations may also be prepared from the solid dispersion according to the invention in order to achieve a more controlled release of the active agent in contact with the body fluids in the gastro intestinal tract, and to provide a substantial constant and effective level of the active agent in the gastric juice. The solid dispersion of the present invention may be embedded for this purpose in a polymer matrix of a biological degradable polymer, a water soluble polymer or a mixture of both, and optionally suitable surfactants. Embedding can mean in this context the incorporation of micro-particles in a matrix of polymers. Modified release formulations can also be obtained through encapsulation of dispersed micro-particles or emulsified micro-droplets via known dispersion or emulsion coating technologies.

The pharmaceutical composition facilitates administration of the compound to a mammal, preferably to a human. Idelalisib can be used singly or in combination with one or more therapeutic agents as components of mixtures.

Amorphous Idelalisib allows the preparation of pharmaceutical compositions which are bioequivalent to the pharmaceutical composition approved with the US NDA no. 205858.

### EXAMPLES

In the following, the present invention will further be described by the way of non-limiting examples.

### Methods of Measurement and Analyses

### X-Ray Powder Diffraction (XRPD)

XRPD diffractograms were obtained with an X'Pert PRO diffractometer (PANalytical, Almelo, The Netherlands) equipped with a theta/theta coupled goniometer in transmission geometry, programmable XYZ stage with well plate holder, Cu K-alpha radiation source (wavelength 0.15418 nm) with a focusing mirror, a 0.5° divergence slit, a 0.02° soller slit collimator and a 0.5° anti-scattering slit on the incident beam side, a 2 mm anti-scattering slit, a 0.02° soller slit collimator, a Ni-filter and a solid state PIXcel detector on the diffracted beam side. The diffractogram was recorded at room temperature (25°C) at a tube voltage of 40 kV, tube current of 40 mA, applying a step size of 0.013° 2-Theta with 40 sec per step in the angular range of 2° to 40° 2-Theta. A typical precision of the 2-Theta values is in the range of ± 0.2° 2-Theta. Thus, a diffraction peak that appears for example at 9.4° 2-Theta can appear between 9.2 and 9.6° 2-theta on most X-ray diffractometers under standard conditions.

### Thermogravimetric/Differential Thermal Analysis (TG/DTA)

Each sample of material was placed into an aluminum pan and loaded into a simultaneous thermogravimetric/differential thermal analyzer (TGA/DSC1 with STARe software, Mettler-Toledo LLC, Columbus, USA) and held at 30°C for one minute. The sample was then heated under nitrogen (flow rate: 100 cm3/min) at a rate of 10°C/min from 30°C to 300°C during which time the change in sample weight was recorded along with any differential thermal events (DTA). The calibration standards were indium and tin.
1H Nuclear Magnetic Resonance Spectroscopy (NMR)
NMR analysis was carried out on a 500MHz instrument (Bruker Biospin AG, Fallanden, Switzerland) in CDC13 as internal standard.

### Preparation of Solvates of Idelalisib

### Example 1: tert-butanol solvate of idelalisib

To amorphous idelalisib (180 mg) were added 500 µL of tert-butanol/water 95:5 (v/v) at 23°C. The amorphous material dissolved. After 5 min of stirring, solids began to precipitate. The suspension was stirred at 23°C for 5 days, followed by centrifugation and separation of the precipitate. The solid was then dried, leading to a free flowing white powder (120 mg, 55%) being a crystalline tert-butanol solvate of idelalisib containing water. Ratio idelalisib : tert-butanol : water was about 1 : 0.5 : 3.

The peak list corresponding to the XRPD pattern of crystalline tert-butanol solvate of idelalisib shown in Fig. 3 is given in the following table:

| | | | | | | | | | |
|---|---|---|---|---|---|---|---|---|---|
| Pos. [°2Theta] | 9.4 | 10.3 | 11.6 | 12.6 | 15.8 | 16.0 | 17.2 | 18.9 | 20.7 |
| Rel. Int. [%] | 83 | 62 | 32 | 77 | 32 | 24 | 36 | 82 | 79 |
| | | | | | | | | | |
| 22.2 | 22.8 | 23.0 | 23.1 | | 28.8 | 38.6 | | | |
| 26 | 100 | 26 | 24 | | 26 | 25 | | | |

Further, the tert-butanol solvate of idelalisib has a TG/DTA thermogram as illustrated in Fig. 4.

### Example 2: Tetrahydrofuran solvate of idelalisib

Amorphous idelalisib (50 mg) was dissolved at 25°C in 200 µL tetrahydrofuran. After a few minutes of stirring, solids began to precipitate. The suspension was stirred at 25°C for 5 days, followed by centrifugation and separation of the precipitate. The solid was then dried, leading to crystalline tetrahydrofuran solvate of idelalisib.

The peak list corresponding to the XRPD pattern of crystalline tetrahydrofuran solvate of idelalisib shown in Fig. 5 is given in the following table:

| | | | | | | | | | | | |
|---|---|---|---|---|---|---|---|---|---|---|---|
| Pos. [°2Theta] | 7.5 | 7.6 | | 9.8 | 10.9 | 15.7 | | 17.5 | 18.1 | 18.3 | 18.7 |
| Rel. Int. [%] | 33 | 24 | | 31 | 53 | 43 | | 89 | 66 | 30 | 59 |
| | | | | | | | | | | | |
| 19.4 | 20.5 | | 21.5 | | 26.4 | | 27.7 | | 28.1 | | |
| 73 | 40 | | 100 | | 34 | | 26 | | 26 | | |

Further, the tetrahydrofuran solvate of idelalisib had a TG/DTA thermogram as illustrated in Fig. 6.

### Example 3: N,N-dimethylformamide solvate of idelalisib

### Route A

Form I idelalisib (50 mg) was added to N,N-dimethylformamide (DMF) at 5°C until undissolved solids remained. The suspension was stirred at 5°C for 5 days, followed by centrifugation and separation of the precipitate. The solid was then dried, leading to crystalline N,N-dimethylformamide solvate of idelalisib. 1H NMR and TG/DTA evaluation showed the solvate to be a 1:1 DMF idelalisib solvate.

The peak list corresponding to the XRPD pattern of crystalline DMF solvate of idelalisib shown in Fig. 7 is given in the following table:

| | | | | | | | | | | | |
|---|---|---|---|---|---|---|---|---|---|---|---|
| Pos. [°2Theta] | 10.8 | 11.2 | | 11.8 | 17.5 | 18.2 | | 18.3 | 19.8 | 20.1 | 21.3 |
| Rel.Int. [%] | 41 | 43 | | 75 | 47 | 91 | | 95 | 32 | 100 | 44 |
| | | | | | | | | | | | |
| 22.6 | 23.6 | | 25.4 | | 28.4 | | 28.7 | | | | |
| 30 | 72 | | 50 | | 33 | | 31 | | | | |

Further, the DMF solvate of idelalisib had a TG/DTA thermogram as illustrated in Fig. 8.

Form I idelalisib (50 mg) was added to 100 µL N,N-dimethylformamide (DMF) at 5°C. The solution was then added to n-heptane (1 mL) as anti-solvent at 5°C. Since a solid did not precipitate, the solution was sonicated at 30% intensity using a Cole-Parmer 130W ultrasonic processor, stored at 5°C for 16 h and the solvent was then removed by evaporation, leading to crystalline DMF solvate (1:1) which had an XRPD and a TG/DTA substantially as shown in Figs. 7 and 8.

### Preparation of Amorphous Idelalisib

### Example 4: Amorphous Idelalisib

### a) By Precipitation

33 mg of idelalisib tert-butanol solvate of Example 1 were dissolved in 60 µL ethanol at 40°C. The solution was centrifuged, filtered and the filtrate added to 600 µL water, leading immediately to the precipitation of a solid. After stirring for 5 min, the solid was separated from the solvent by centrifuge filtration. Analysis by XRPD showed the solid to be pure amorphous idelalisib, with no noticeable peaks of a crystalline form being present, as illustrated in Fig. 1.

Pure amorphous idelalisib had a solubility in water at 25°C of about 415 mg/L.

### b) By Lyophilization

Essentially pure amorphous form of idelalisib was prepared according to the following protocol: 25 mg of THF solvate of idelalisib (prepared according to Example 2) were dissolved in 1.0 mL of 1,4-dioxane at a temperature of from 40 to 50°C. The solution was sonicated (2 to 5 min in a VWR Ultrasonic Cleaner apparatus) to allow faster solid dissolution. The solution was filtered through a 0.2 µm filter into a round-bottomed flask and rotated within a Dewar flask containing a mixture of liquid nitrogen and acetone, forming a frozen film on the inside of the flask. The flask was lyophilized (plate temperature 20°C, pressure 0.08 mbar) during 18 hours, yielding essentially pure amorphous form of idelalisib. The XRPD pattern of the obtained amorphous idelalisib is shown in Fig. 1.

### Example 5: Compression Stability of Pure Amorphous Idelalisib

50 mg of pure amorphous idelalisib obtained as disclosed in Example 4b) were added to a KBr pellet die and compressed for 4.5 days at 740 MPa. The resultant solid disc was removed from the press and analysed by XRPD, showing no change in the amorphous phase, as illustrated in Fig. 2.

### Example 6: Thermal and Humidity Stability of Pure Amorphous Idelalisib

### a) 98% Relative Humidity

50 mg of pure amorphous idelalisib obtained as disclosed in Example 4b) were exposed to 98% relative humidity at 25°C for 5 days. XRPD analysis provided an XRPD pattern substantially as shown in Fig. 1, showing no change of the amorphous form.

### b) 40°C/75% Relative Humidity

50 mg of pure amorphous idelalisib obtained as disclosed in Example 4b) were exposed to 75% relative humidity at 40°C for 5 days. XRPD analysis provided an XRPD pattern substantially as shown in Fig. 1, showing no change of the amorphous form.

### c) 120°C/50% Relative Humidity

50 mg of pure amorphous idelalisib obtained as disclosed in Example 4b) were thermally stressed at 120°C and 50% relative humidity for 5 days in a sealed vial. XRPD analysis provided an XRPD pattern substantially as shown in Fig. 1, showing no change of the amorphous form.

## Claims

1. Amorphous idelalisib having no noticeable peak in an X-ray powder diffractogram, when measured at a temperature of 25°C with Cu K-alpha radiation.

2. Amorphous idelalisib according to claim 1 wherein at most 300 ppm of ICH class 2 solvent are present, preferably at most 100 ppm and most preferably at most 10 ppm.

3. A process of forming amorphous idelalisib of any one of claims 1 to 2 comprising the steps of
(i) providing an idelalisib solvate,
(ii) dissolving the idelalisib solvate of step (i) in a solvent, and
(iii) removing the solvent.

4. Process of forming amorphous idelalisib according to claim 3, wherein step (iii) is carried out by adding the solution of step (ii) into water and filtrating the precipitate.

5. Process of forming amorphous idelalisib according to claim 4, wherein the solvent in step (ii) is selected from ethanol, methanol, dioxane, tetrahydrofurane and N,N-dimethylformamide.

6. Process of forming amorphous idelalisib according to claim 3, wherein step (iii) is carried out by spray or freeze drying the solution of step (ii).

7. Process of forming amorphous idelalisib according to claim 3, wherein the solvent in step (ii) is selected from dioxane, tetrahydrofuran, ethanol and methanol.

8. A solvate of idelalisib and a solvent, wherein the solvent is selected from *tert*-butanol, N,N-dimethylformamide and tetrahydrofuran.

9. Crystalline idelalisib *tert*-butanol solvate **characterized by** data selected from one or more of the following: an X-ray powder diffraction pattern with characteristic peaks at 9.4±0.2, 18.9±0.2, 22.8±0.2° 2-Theta when measured at a temperature of 25 °C with Cu K-alpha; an X-ray powder diffraction pattern substantially as depicted in Fig. 2; and by a combination of these data.

10. Crystalline idelalisib *N,N*-dimethylformamide solvate **characterized by** data selected from one or more of the following: an X-ray powder diffraction pattern with characteristic peaks at 11.8±0.2, 18.3±0.2, 20.1±0.2° 2-Theta when measured at a temperature of 25 °C with Cu-Kalpha; an X-ray powder diffraction pattern substantially as depicted in Fig. 3; and by a combination of these data.

11. Crystalline idelalisib tetrahydrofuran solvate **characterized by** data selected from one or more of the following: an X-ray powder diffraction pattern with characteristic peaks at 17.5±0.2, 19.4±0.2, 21.5±0.2° 2-Theta when measured at a temperature of 25 °C with Cu-Kalpha; an X-ray powder diffraction pattern substantially as depicted in Fig. 4; and by a combination of these data.

12. Process for the preparation of idelalisib solvates of claim 8 to 11 comprising the steps of
(I) providing a solution of idelalisib in the corresponding solvent,
(II) optionally adding seed crystals,
(III) stirring the solution, or performing one or more of temperature cycling, crash cooling, evaporation, or anti-solvent addition to form a slurry,
(IV) isolating the crystalline solvate in solid form, and
(V) optionally drying the crystalline solvate.

13. Use of a crystalline idelalisib solvate for the production of pure and stable amorphous idelalisib.

14. Process for the purification of crude idelalisib, comprising the steps of:
(I) providing a solution of crude idelalisib in an organic solvent,
(II) optionally adding seed crystals, and
(III) stirring the solution, or performing one or more of temperature cycling, crash cooling, evaporation, or anti-solvent addition to form a slurry,
(IV) isolating the crystalline solvate in solid form,
(V) dissolving the crystalline solvate of idelalisib of step (IV) in a solvent,
(VI) removing the solvent, and
(VII) optionally drying the product obtained in step (VI), in order to yield amorphous, preferably pure amorphous idelalisib.

15. A pharmaceutical composition comprising amorphous idelalisib or a solvate of idelalisib of any one of claims 1 to 14, and one or more pharmaceutically acceptable excipients.
